# EUROPÄISCHE PATENTSCHRIFT

(11) **EP 0 825 181 B1**
(45) Veröffentlichungstag und Bekanntmachung des Hinweises auf die Patenterteilung: **24.11.1999**
(21) Anmeldenummer: 97113724.5
(22) Anmeldetag: 08.08.1997
(51) Int. Cl.: C07D 209/86

(54) **Verfahren zur Herstellung von Carbazol**
Process of preparation of carbazole
Procédé pour la préparation de carbazole

(30) Priorität: 21.08.1996 DE 19633609
(43) Veröffentlichungstag der Anmeldung: 25.02.1998
(73) Patentinhaber: BAYER AG, 51368 Leverkusen (DE)
(72) Erfinder: Buysch, Hans-Josef, Dr., 47809 Krefeld (DE); Langer, Reinhard, Dr., 47800 Krefeld (DE); Notheis, Ulrich, Dr., 41539 Dormagen (DE); Klausener, Alexander, Dr., 50670 Köln (DE)

(56) Entgegenhaltungen:
- US-A- 2 921 942
- US-A- 3 041 349
- US-A- 3 085 095

## Beschreibung

Die vorliegende Erfindung betrifft ein Verfahren zur Herstellung von Carbazol durch Dehydrierung bzw. aminierende Dehydrierung der weiter unten genannten Verbindungen der Formeln (I) bis (VIII) in Gegenwart von Iridium-Katalysatoren oder Iridium-enthaltenden Katalysatoren auf Trägern.

Carbazol ist ein Ausgangsprodukt zur Herstellung von Farbstoffen, Polymeren und Insektiziden (Ullmann's Encyclopedia, 5. Ed., Vol. A5, S. 59-60).

Es ist bekannt, daß Carbazol durch Dehydrierung bzw. aminierende Dehydrierung von Verbindungen der weiter unten genannten Formeln (I) bis (VIII), in denen Teile des Carbazol-Gerüstes bereits vorgebildet sind, mit Hilfe von Platin-Katalysatoren hergestellt werden kann. Nach US 2.921.942 wird hierbei Platin auf Al₂O₃ oder auf SiO₂ als Träger eingesetzt. Solche Katalysatoren führen bei annehmbar hohen Umsätzen zu guten Ausbeuten an Carbazol, werden aber bereits nach kurzem Gebrauch von nur wenigen zehn Stunden auch bei so niedrigen Belastungen wie 0,1 g Diphenylamin pro ml Katalysator und Stunde (g/ml · h) oder sogar geringeren Belastungen desaktiviert. Dadurch wird die Produktivität des Katalysators, nämlich die pro Stunde und pro ml Katalysator gebildete Menge an Carbazol, auch bei hohen Umsätzen des eingesetzten Diphenylamins so gering, daß man für technische Zwecke große Katalysatormengen und voluminöse Reaktoren benötigt. Längere Standzeiten zwischen den Katalysatorregenerierungen, nämlich solche von mehreren zehn Stunden, erhält man, wenn als Träger für den Platin-Katalysator Magnesiumoxid gewählt wird (US 3.041.349). Allerdings ist die Lebensdauer dieses Katalysators offenbar nur wenig länger als insgesamt etwa 150 bis 200 Stunden (Tab. II in US' 349), denn nach einer etwas längeren Standzeit als in obiger US' 942, nämlich einer solchen von ca. 60 bzw. 62 Stunden (berechnet nach den Angaben von zugeführtem Diphenylamin (DPA) in US' 349) zwischen je zwei Regenerietungen, sinkt die Zeit bis zur nächsten Regenerierung auf ca. 17 Stunden, was zweifelsohne die baldige völlige Unbrauchbarkeit dieses Katalysators anzeigt. Auch hier liegt die Belastung nur etwa bei 0,1 g/ml · h. Katalysatoren auf der Basis von Chromoxid gemäß US 3.085.095 werden gleichfalls rasch unbrauchbar, wenn sie auch etwas höhere Belastungen vertragen.

Die Aufgabe der vorliegenden Erfindung war demnach die Entwicklung von Katalysatoren, die bei hohen Belastungen gute Umsätze und Ausbeuten an Carbazol ergeben und hohe Standzeiten zwischen zwei Regenerierungen und insgesamt eine lange Lebensdauer aufweisen. Diese Aufgabe konnte gelöst werden, wenn man Iridium-Katalysatoren oder Iridium-enthaltende Katalysatoren, in denen neben dem Iridium andere Metalle der Platinmetallgruppe eingesetzt werden, einsetzt.

Die Erfindung betrifft demnach ein Verfahren zur Herstellung von Carbazol durch Dehydrierung von Verbindungen der Formeln 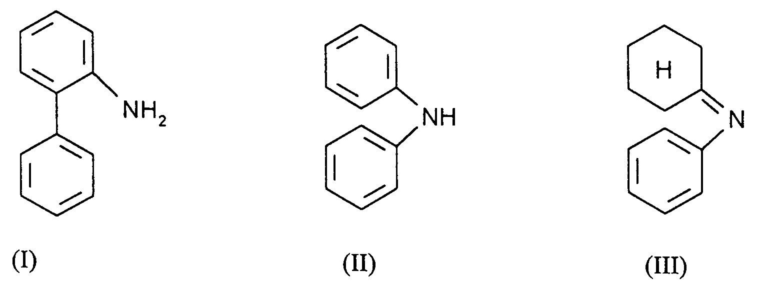 oder durch aminierende Dehydrierung von Verbindungen der Formeln 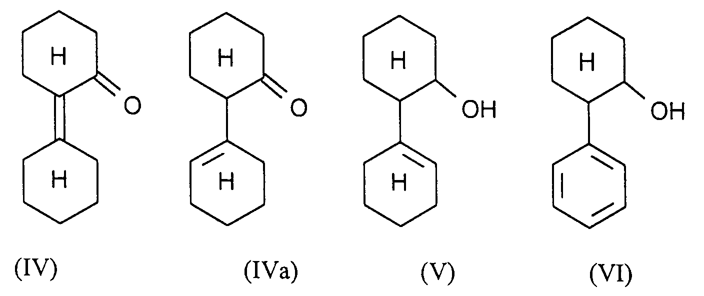 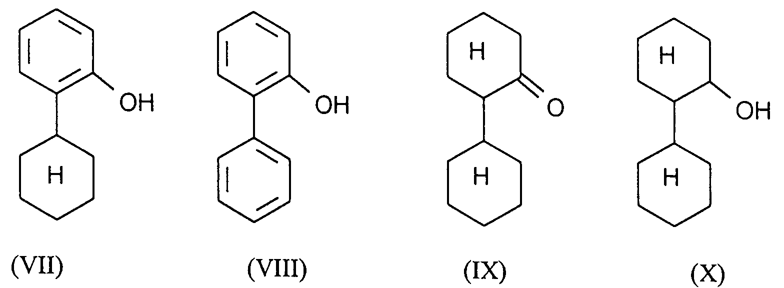 bei erhöhter Temperatur an einem Edelmetall-Katalysator, das dadurch gekennzeichnet ist, daß an einem Iridium-Katalysator oder an einem Iridium-enthaltenden Katalysator bei 300 bis 600°C in Gegenwart von Wasserstoff gearbeitet wird.

Geeignete Ausgangsprodukte für das erfindungsgemäße Verfahren sind o-Phenylanilin (I), Diphenylamin (II), Cyclohexylidenanilin (III; die aus Anilin und Cyclohexanon gebildete Schiff'sche Base = Anil), o-Cyclohexyliden-cyclohexanon (IV; Dianon) und sein Tautomeres (IVa), o-Cyclohexyl-cyclohexanon (IX), o-Cyclohexenyl-cyclohexanol (V), o-Phenyl-cyclohexanol (VI), o-Cyclohexylphenol (VII), o-Phenyl-phenol (VIII) und o-Cyclohexyl-cyclohexanol (X), bevorzugt Diphenylamin, Anil, Dianon, o-Cyclohexylphenol und o-Phenyl-phenol, besonders bevorzugt Diphenylamin.

Die erfindungsgemäßen Katalysatoren enthalten Iridium auf Trägern mit einem Gehalt an Ir von 0,01 bis 10 Gew.-%, bevorzugt 0,1 bis 6 Gew.-%, besonders bevorzugt 0,1 bis 4 Gew.-%, ganz besonders bevorzugt 0,1 bis 2 Gew.-%, bezogen auf das Gesamtgewicht des Katalysators. Erfindungsgemäß können weiterhin Ir enthaltende Katalysatoren auf Trägern eingesetzt werden, die neben dem Gehalt an Ir ein weiteres Metall aus der Gruppe der Platinmetalle enthalten, bevorzugt Rh, Pd oder Pt, besonders bevorzugt Pt enthalten, bei denen die Summe der Edelmetalle 0,01 bis 10 Gew.-%, bevorzugt 0,1 bis 6 Gew`-%, besonders bevorzugt 0,1 bis 4 Gew.-%, ganz besonders bevorzugt 0,1 bis 2 Gew.-%, bezogen auf das Gesamtgewicht des Katalysators, beträgt; das Gewichtsverhältnis der Ir zu dem anderen Platingruppenmetall beträgt Ir : anderes Metall = 0,2 bis 5 : 1, bevorzugt 0,5 bis 2 : 1.

Die Edelmetalle werden in an sich üblicher Weise, beispielsweise durch Tränken oder Sprühen ihrer löslichen Verbindungen auf den Träger aufgebracht und dort durch Fällen, Calcinieren und Reduzieren zu den Metallen fixiert. Hierfür geeignete Methoden sind dem Fachmann bekannt und beispielsweise beschrieben in P. N. Rylander, Catalytic Hydrogenation over Platin Metals, Academic Press 1967, S. 25 bis 29 oder P. N. Rylander, Hydrogenation Methods, Academic Press, 1985.

Als lösliche Verbindungen von Iridium und den anderen Platingruppenmetallen kommen einfache oder komplexe salzartige Verbindungen in Frage, beispielsweise die Halogenide, Nitrate, Acetate, Acetylacetonate, Carbonyl-Komplexe, Phosphin-oder Phosphit-Komplexe sowie Ammoniak- und Amin-Komplexe. Zur Aufbringung auf den Träger können viele gängige organische Lösungsmittel oder Wasser sowie Gemische daraus eingesetzt werden; Beispiele für organische Lösungsmittel sind niedere Alkohole, Nitrile, Ketone, Chlorkohlenwasserstoffe, Ester und Amide. Von den genannten Lösungsmittelgruppen werden vorwiegend die niederen Glieder ihrer homologen Reihen, beispielsweise mit 1 bis 4 C-Atomen eingesetzt. Wegen der unproblematischen Verarbeitung wird Wasser den organischen Lösungsmitteln vorgezogen. Die Träger können vor oder nach dem Auftragen der Edelmetall-Lösungen mit alkalischen Verbindungen behandelt werden; ebenso kommt eine Behandlung mit alkalischen Verbindungen sowohl vor der Reduktion des Metallsalzes als auch danach in Frage. Hierfür geeignete alkalische Verbindungen sind (Erd)Alkalimetallhydroxide, wie NaOH, KOH, Ca(OH)₂ oder Ba(OH)₂, in wäßriger Lösung oder Alkalicarbonate, wie Na₂CO₃ oder K₂CO₃, ebenfalls in wäßriger Lösung. Bei dieser Behandlung werden die Edelmetalle in bekannter Weise als Hydroxide oder Oxide ausgefällt und so auf dem Träger fixiert. Die fixierten Edelmetalloxide bzw. -hydroxide werden sodann mit Wasserstoff zum elementaren Metall reduziert. Diese Behandlung mit Wasserstoff kann grundsätzlich in der Anfahrphase des erfindungsgemäßen Verfahrens durchgeführt werden; in bevorzugter Weise wird aber eine besondere Aktivierungsphase für den Katalysator vorgesehen, innerhalb derer er durch H₂-Behandlung in die aktive metallische Form übergeführt wird.

Geeignete Träger für die erfindungsgemäß eingesetzten Edelmetall-Katalysatoren sind Oxide und Oxidhydrate von Elementen aus der II. bis VIII. Gruppe des Periodensystems der Elemente (Mendelejew). Als Elemente seien beispielsweise genannt: Mg, Ca, Ba, Zn, Al, Ga, La, Si, Sn, Ti, Zr, Nb, Ta, Cr, Fe, Co, Ni, bevorzugt Mg, Ca, Zn, Al, Si, Sn, Ti, Zr, Nb, Cr, besonders bevorzugt Mg, Zn, Al, Si, Ti, Zr. Weitere brauchbare Träger für erfindungsgemäß einsetzbare Katalysatoren sind Schichtsilikate, wie Bentonit und Montmorillonit, ferner Mischoxide aus den obigen Elementen wie beispielsweise Al/Si, Ti/Zr, Nb/Ta, Mg/Ca, Ca/Si, Mg/Si, Zn/Si, Zn/Ti, Al/Ti, die u.a. auf dem Weg über mechanische Vermischungen, gemeinsame Fällungen von Salzen oder über Cogele aus Salzen oder Alkoxiden hergestellt werden können, wie dies dem Fachmann bekannt ist (D.A. Ward et al., Ind. Eng, Chem. Res. 34 (1995), 421 bis 433; W.F. Maier et al., in J.F. Harrod and R.M. Laine, Applications of Organometallic Chemistry in the Preparation and Processing of Advanced Materials, Kluwer Academic Publishers 1995, Seite 27 bis 46).

Im erfindungsgemäßen Verfahren wird dem Einsatzstoff der Formel (I) bis (X) wenigstens eine geringe Menge an Wasserstoff zugesetzt, obwohl es sich erfindungsgemäß um eine Dehydrierung bzw. eine aminierende Dehydrierung handelt, bei der grundsätzlich Wasserstoff entsteht. Diese zugesetzte Menge Wasserstoff beträgt 0,01 bis 300 Mol H₂, bevorzugt 0,1 bis 200 Mol H₂, besonders bevorzugt 0,2 bis 150 Mol H₂ pro Mol der zu dehydrierenden bzw. aminierend dehydrierenden Verbindung.

Während bei den unter Bildung von Carbazol zu dehydrierenden Verbindungen der Formeln (I) bis (III) die Stickstoff-Funktion im Edukt bereits vorliegt, muß sie in Edukten der Formeln (IV) bis (X) erst durch Austausch der Sauerstoff-Funktion mit Hilfe von Ammoniak hergestellt werden. Der hierzu benötigte gleichzeitige Einsatz von Ammoniak beträgt 1 bis 50 Mol NH₃, bevorzugt 1,5 bis 40 Mol NH₃, besonders bevorzugt 2 bis 30 Mol NH₃ pro Mol der aminierend zu dehydrierenden Verbindung der Formeln (IV) bis (X).

Weiterhin wurde gefunden, daß im erfindungsgemäßen Verfahren die Gegenwart von Wasser zulässig ist, wenngleich Wasser für die Durchführung der erfindungsgemäßen Reaktion auch nicht erforderlich ist. Die Wassermenge beträgt daher allgemein 0 bis 10 Mol H₂O pro Mol der zu dehydrierenden oder aminierend dehydrierenden Verbindung der Formeln (I) bis (X), wobei die Untergrenze Null die Abwesenheit von Wasser bedeutet. Für den Fall der Anwesenheit von Wasser kann dies ein Bereich von 0,05 bis 10 Mol H₂O, bevorzugt 0,1 bis 8 Mol, besonders bevorzugt 0,15 bis 5 Mol H₂O pro Mol der Verbindung (I) bis (X) sein.

Das erfindungsgemäße Verfahren wird im Temperaturbereich von 300 bis 600°C, bevorzugt 350 bis 580°C, besonders bevorzugt 380 bis 570°C und in einem Druckbereich von 0,5 bis 10 bar, bevorzugt 0,7 bis 8 bar, besonders bevorzugt 0,8 bis 5 bar durchgeführt.

### Beispiel 1 (Herstellung des Katalysators)

2 l γ-Al₂O₃ (Typ SPH 501 der Fa. Rhone-Poulenc; Kugeln von 3 bis 4 mm ⌀ ) wurden mit 665 ml einer wäßrigen Lösung, enthaltend 8 g Pt und 8 g Ir (32 g H₂PtCl₆ als 25 %ige Lösung und 15,4 g IrCl₄), getränkt und im Trockenschrank 24 h bei 150°C getrocknet. Danach wurde mit 665 g wäßriger KOH-Lösung, enthaltend 34 g KOH, getränkt und wiederum getrocknet. Der Katalysator enthielt 0,4 Gew.-% Ir und 0,4 Gew.-% Pt.

### Beispiel 2 (Herstellung des Katalysators)

Analog Beispiel 1 wurde ein Katalysator hergestellt, der kein Pt, sondern 1,2 % Iridium enthielt.

### Beispiel 3 (Vergleich)

Analog Beispiel 1 wurde ein Katalysator hergestellt, der 1,2 % Pt enthielt.

### Beispiel 4

12 ml des Katalysators nach Beispiel 1 wurden in ein Quarzrohr von 1,6 mm ⌀ gefüllt, bei 250°C mit Wasserstoff reduziert und dann bei Normaldruck mit 0,5 g/ml · h DPA und 100 Mol H₂/Mol DPA belastet. Über lange Zeit erhielt man eine stabile Umsetzung von DPA zu Carbazol bei 35 bis 45 % und hoher Selektivität zwischen 80 und 90 %. Nach 1000 h und kontinuierlicher Anhebung der Temperatur ab 350°C betrug der DPA-Umsatz bei 450°C ohne Zwischenregenerierung immer noch 38 % bei einer Selektivität von 87 %. Anzeichen einer beginnenden Desaktivierung waren nicht zu erkennen.

### Beispiel 5

Der Katalysator aus Beispiel 2 ergab unter den Bedingungen des Beispiels 4 einen Umsatz an DPA von 53 % und eine Selektivität von 59 %.

### Beispiel 6 (Vergleich)

Der Katalysator aus Beispiel 3 ergab unter den Bedingungen des Beispiels 4 einen Umsatz von 49 % und eine Selektivität von 38 %.

## Patentansprüche

1. Verfahren zur Herstellung von Carbazol durch Dehydrierung von Verbindungen der Formeln 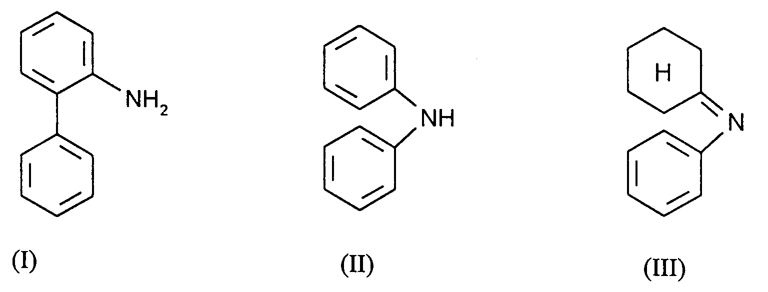 oder durch aminierende Dehydrierung von Verbindungen der Formeln 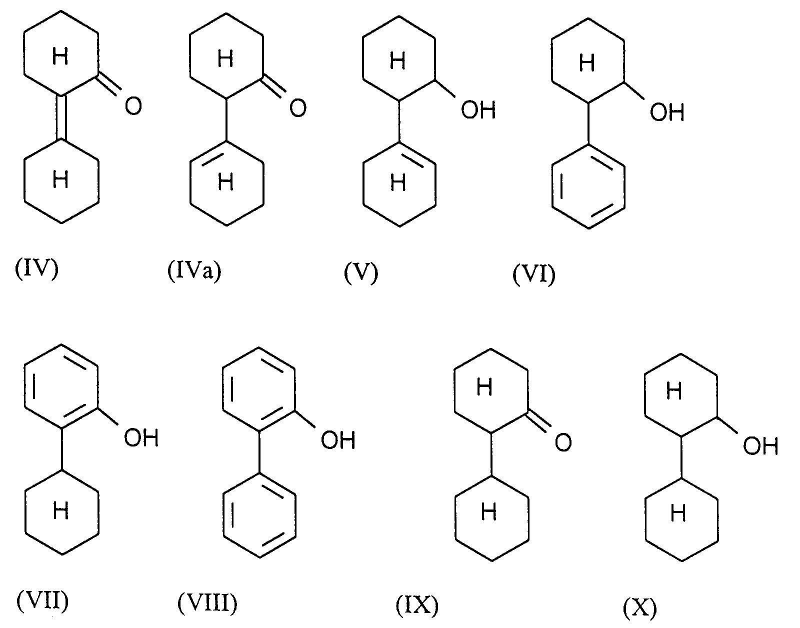 bei erhöhter Temperatur an einem Edelmetall-Katalysator, dadurch gekennzeichnet, daß an einem Iridium-Katalysator oder an einem Iridium enthaltenden Katalysator bei 300 bis 600°C in Gegenwart von Wasserstoff gearbeitet wird.

2. Verfahren nach Anspruch 1, dadurch gekennzeichnet, daß der Katalysator einen Gehalt an Iridium von 0,01 bis 10 Gew.-%, bevorzugt 0,1 bis 6 Gew.-%, besonders bevorzugt 0,1 bis 4 Gew.-%, ganz besonders bevorzugt 0,1 bis 2 Gew.-%, bezogen auf das Gesamtgewicht des Katalysators, hat.

3. Verfahren nach Anspruch 1, dadurch gekennzeichnet, daß der Katalysator ein Iridium enthaltender Katalysator ist, wobei neben dem Iridium ein weiteres Metall aus der Gruppe der Platinmetalle, bevorzugt Rhodium, Palladium, Platin, besonders bevorzugt Platin, vorliegt, die Summe der Edelmetalle 0,01 bis 10 Gew.-%, bevorzugt 0,1 bis 6 Gew.-%, besonders bevorzugt 0,1 bis 4 Gew.-%, ganz besonders bevorzugt 0,1 bis 2 Gew.-%, bezogen auf das Gesamtgewicht des Katalysators, beträgt und das Gewichtsverhältnis des Iridium zu dem anderen Platingruppenmetall Ir : anders Metall = 0,2 bis 5 : 1, bevorzugt 0,5 bis 2 : 1 beträgt.

4. Verfahren nach Anspruch 1, dadurch gekennzeichnet, daß als zu dehydrierende oder aminierend zu dehydrierende Verbindung die der Formel (II), (III), (IV), (VII) oder (VIII), bevorzugt die der Formel (II) eingesetzt wird.

5. Verfahren nach Anspruch 1, dadurch gekennzeichnet, daß 0,01 bis 300 Mol H₂, bevorzugt 0,1 bis 200 Mol H₂, besonders bevorzugt 0,2 bis 150 Mol H₂ pro Mol der zu dehydrierenden bzw. aminierend dehydrierenden Verbindung eingesetzt werden.

6. Verfahren nach Anspruch 1, dadurch gekennzeichnet, daß für den Fall der aminierenden Dehydrierung 1 bis 50 Mol NH₃, bevorzugt 1,5 bis 40 Mol NH₃, besonders bevorzugt 2 bis 30 Mol NH₃ pro Mol der zu aminierend dehydrierenden Verbindung eingesetzt werden.

7. Verfahren nach Anspruch 1, dadurch gekennzeichnet, daß bei 350 bis 580°C, bevorzugt 380 bis 570°C gearbeitet wird.

8. Verfahren nach Anspruch 1, dadurch gekennzeichnet, daß bei 0,5 bis 10 bar, bevorzugt bei 0,7 bis 8 bar, besonders bevorzugt bei 0,8 bis 5 bar gearbeitet wird.

## Claims

1. Process for the preparation of carbazole by dehydrogenation of a compound of the formula 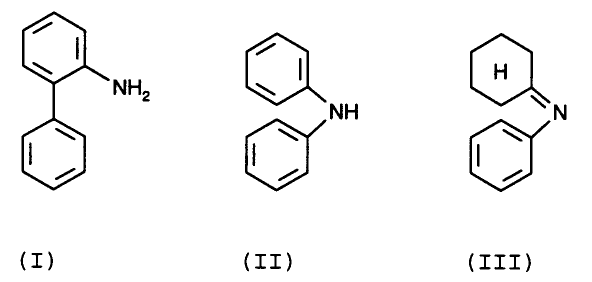 or by aminating dehydrogenation of the compound of the formula 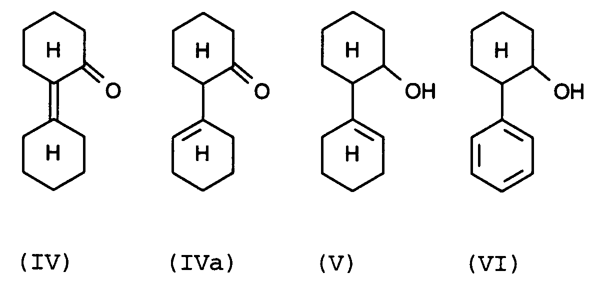 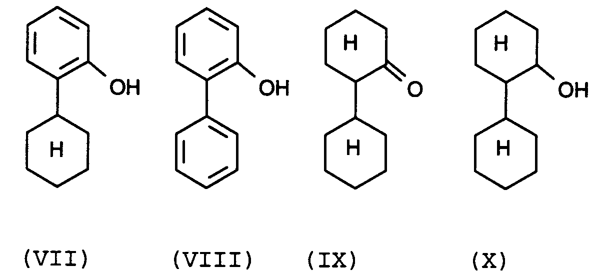 at elevated temperature over a noble metal catalyst, characterized in that an iridium catalyst or an iridium-containing catalyst is used at 300 to 600 C in the presence of hydrogen.

2. Process according to Claim 1, characterized in that the catalyst has a content of iridium of 0.01 to 10 % by weight, preferably 0.1 to 6 % by weight, particularly preferably 0.1 to 4 % by weight, especially preferably 0.1 to 2 % by weight, based on the total weight of the catalyst.

3. Process according to Claim 1, characterized in that the catalyst is an iridium-containing catalyst in which, in addition to the iridium, another metal from the group of platinum metals, preferably rhodium, palladium or platinum, particularly preferably platinum, is present, the sum of the noble metals being 0.01 to 10 % by weight, preferably 0.1 to 6 % by weight, particularly preferably 0.1 to 4 % by weight, especially preferably 0.1 to 2 % by weight, based on the total weight of the catalyst, and the weight ratio of iridium to the other platinum group metal Ir : other metal = 0.2 to 5 : 1, preferably 0.5 to 2 : 1.

4. Process according to Claim 1, characterized in that the compound of the formula (II), (III), (IV), (VII) or (VIII), preferably the compound of the formula (II), is employed as the compound to be dehydrogenated or subjected to aminating dehydrogenation.

5. Process according to Claim 1, characterized in that 0.01 to 300 mol of H₂, preferably 0.1 to 200 mol of H₂, particularly preferably 0.2 to 150 mol of H₂, are employed per mole of the compound to be dehydrogenated or subjected to aminating dehydrogenation.

6. Process according to Claim 1, characterized in that, in the event of aminating dehydrogenation, 1 to 50 mol of NH₃, preferably 1.5 to 40 mol of NH₃, particularly preferably 2 to 30 mol of NH₃, per mole of the compound to be subjected to aminating dehydrogenation are used.

7. Process according to Claim 1, characterized in that the reaction is carried out at 350 to 580 C, preferably 380 to 570 C.

8. Process according to Claim 1, characterized in that the reaction is carried out under 0.5 to 10 bar, preferably under 0.7 to 8 bar, particularly preferably under 0.8 to 5 bar.

## Revendications

1. Procédé pour la préparation du carbazole par déshydrogénation de composés de formules 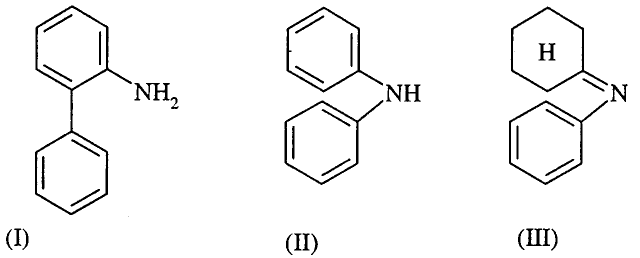 ou par déshydrogénation aminante de composés de formules 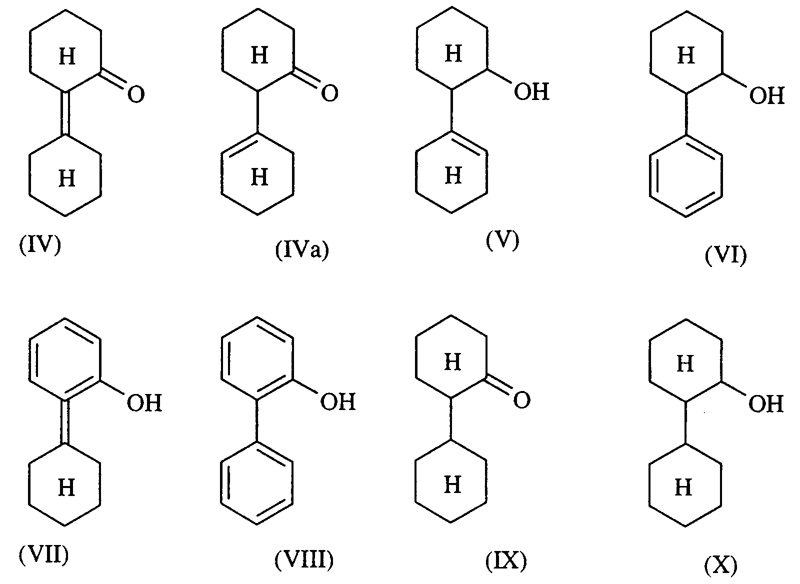 à température élevée sur un catalyseur à base de métaux nobles, caractérisé en ce que l'on opère sur un catalyseur consistant en iridium ou contenant de l'iridium à des températures de 300 à 600°C en présence d'hydrogène.

2. Procédé selon la revendication 1, caractérisé en ce que le catalyseur contient 0,01 à 10 % en poids, de préférence 0,1 à 6 % en poids, plus spécialement 0,1 à 4 % en poids et dans les meilleures conditions 0,1 à 2 % en poids d'iridium par rapport à son poids total.

3. Procédé selon la revendication 1, caractérisé en ce que le catalyseur contient de l'iridium et, en plus de celui-ci, un autre métal du groupe des métaux du platine, de préférence le rhodium, le palladium, le platine, plus spécialement le platine, la somme des métaux nobles représentant 0,01 à 10 %, de préférence 0,1 à 6 %, plus spécialement 0,1 à 4 % et dans les meilleures conditions 0,1 à 2 % du poids total du catalyseur, et les proportions relatives entre l'iridium et l'autre métal du groupe du platine étant : Ir/autre métal = 0,2 à 5:1, de préférence 0,5 à 2:1.

4. Procédé selon la revendication 1, caractérisé en ce que le composé soumis à déshydrogénation ou déshydrogénation aminante est le composé de formule (II), de formule (III), de formule (IV), de formule (VII) ou de formule (VIII) et de préférence le composé de formule (II).

5. Procédé selon la revendication 1, caractérisé en ce que l'on met en oeuvre de 0,01 à 300 mol de H₂, de préférence de 0,1 à 200 mol de H₂, plus spécialement de 0,2 à 150 mol de H₂ par mole du composé soumis à déshydrogénation ou déshydrogénation aminante.

6. Procédé selon la revendication 1, caractérisé en ce que, dans le cas de la déshydrogénation aminante, on met en oeuvre de 1 à 50 mol de NH₃, de préférence de 1,5 à 40 mol de NH₃ et plus spécialement de 2 à 30 mol de NH₃ par mole du composé soumis à la déshydrogénation aminante.

7. Procédé selon la revendication 1, caractérisé en ce que l'on opère à des températures de 350 à 580°C, de préférence de 380 à 570°C.

8. Procédé selon la revendication 1, caractérisé en ce que l'on opère sous une pression de 0,5 à 10 bar, de préférence de 0,7 à 8 bar et plus spécialement de 0,8 à 5 bar.
